# EUROPÄISCHE PATENTANMELDUNG

(11) **EP 0 521 509 A1**
(43) Veröffentlichungstag der Anmeldung: **07.01.1993**
(21) Anmeldenummer: 92111243.9
(22) Anmeldetag: 02.07.1992
(51) Int. Cl.: A61L 15/28, A61L 15/42, A61F 13/15

(54) **Saugfähiges Material und dessen Verwendung**

(30) Priorität: 05.07.1991 DE 4122359
(71) Anmelder: HELEN HARPER HYGIENIC GmbH, D-86911 Diessen (DE)
(72) Erfinder: Putzier, Heiner, Egerstrasse 9, W-8918 Diessen (DE)
(74) Vertreter: Koepe, Gerd L., Dipl.-Chem.

(57) **Zusammenfassung**

Die Erfindung betrifft ein saugfähiges Material aus einem oder mehreren flüssigkeitsdurchlässigen Schichten und einem Saugkörper mit hoher Absorptions- und Retentionskapazität für wäßrige Flüssigkeiten gekennzeichnet durch einen Aufbau aus einer Umhüllung 1 aus einem vliesartigen Stoff aus einem organischen Polymer, einer Tissuezwischenlage 2 aus einem organischen Polymer, einem aus Zellstoff und einem Absorbens für wäßrige Flüssigkeiten bestehenden Saugkörper 3 und einem die Umhüllung stabilisierenden Binder sowie die Verwendung derartiger saugfähiger Materialien für die Herstellung saugfähiger Formkörper, Einlagen und Unterlagen.

Die Erfindung betrifft auch eine ein solches saugfähiges Material in Kombination mit einer flüssigkeitsdichten Umhüllung umfassende Anordnung.

## Beschreibung

Die Erfindung betrifft ein aus einer oder mehreren Schicht(en) aufgebautes saugfähiges Material. Insbesondere betrifft die Erfindung ein mehrschichtiges saugfähiges Material, dessen Bestandteile unter biologischen Bedingungen abbaubar, beispielsweise kompostierbar oder humifizierbar sind. Die Erfindung betrifft darüberhinaus die Verwendung eines derartigen Materials als Einlage für Windeln, Hygieneeinlagen bzw. -unterlagen, Inkontinenzschutzunterlagen und dergleichen sowie Anordnungen, die ein solches saugfähiges Material umfassen.

Auf dem Gebiet der Hygiene- und Inkontinenzartikel sind in jüngerer Zeit saugfähige Materialien entwickelt worden, die sich von früheren bekannten Materialien nicht nur dadurch unterscheiden, daß sie in kürzester Zeit große Mengen an Flüssigkeit absorbieren können, sondern auch dadurch, daß es aufgrund ihres chemisches Aufbaus möglich ist, daß die Materialien diese Flüssigkeit halten, ohne an den betroffenen Körperstellen ein Feuchtigkeitsgefühl zu erzeugen. Sowohl das Absorptionsvermögen als auch das Retentionsvermögen saugfähiger Materialien für die genannten Zwecke wurden sukzessive verbessert. Insbesondere im Bereich von Windein bzw. Windeleinlagen für Babys wurden Materialien entwickelt, die ein weniger häufiges Wickeln der Kleinkinder erlaubten. Dabei wurden in zunehmendem Maße sogenannten Einstück-Systeme (one piece systems) bereitgestellt, die aus saugfähigem Innenkörper und einer meist aus einem synthetischen, wasserabstoßenden Polymeren bestehenden äußeren Hülle bestanden. Diese Systeme erlaubten ein vollständiges Entsorgen des gesamten "Stücks" einschließlich der davon aufgenommenen Fäkalien bzw. Körperflüssigkeiten. Dies wurde weithin auch als eine Verbesserung der Bequemlichkeit in der Babypflege angesehen.

Offensichtlich vernachlässigt wurde in diesem Zusammenhang die Überlegung, daß diese sogenannten "Einstücksysteme" dazu beitrugen, daß die ohnehin steigenden Müllmengen weiter anstiegen. Insbesondere trugen diese Systeme dazu bei, daß der Müll mit Materialien angereichert wurde, die nicht durch natürliche chemische Prozesse abgebaut werden können, sondern mehr oder weniger unverrottbar auf den Mülldeponien lagern oder in aufwendigen pyrolytischen Verfahren (Müllverbrennung) abgebaut werden müssen.

In dem Bestreben, die steigenden Müllmengen nicht noch weiter ansteigen zu lassen, sie möglicherweise sogar zu reduzieren, kam man in jüngerer Zeit wieder auf die bereits früher gebräuchlichen, zwischenzeitlich jedoch als zuwenig bequem empfundenen sogenannten "Zweistücksysteme" (two-piece systems) zurück. Diese bestehen üblicherweise darin, daß ein regelmäßig gewechselter saugfähiger Innenkörper zusammen mit einer mehrmals verwendbaren und auch waschbaren äußeren Hülle verwendet wird. Damit könnte die im Zusammenhang mit Hygieneeinlagen, Windeln, Inkontinenzmaterialien usw. anfallende Abfallmenge in gewissem Umfang reduziert werden.

Zur biologischen Abbaubarkeit (Kompostierbarkeit) der in diesem Zusammenhang vorgeschlagenen "Zweistücksysteme" war jedoch bisher nichts bekannt. In jüngerer Zeit bestand im Zuge der oben bereits angesprochenen Bestrebungen jedoch ein deutlicher Bedarf nach saugfähigen Einlagen für Babywindeln, Hygieneartikeln wie Damenbinden oder Inkontinenzeinlagen und dergleichen, die unter biologischen Bedingungen abbaubar, d. h. kompostierbar oder humifizierbar, sind und die so dazu beitragen, daß die einer Deponie zugeführten Müllmengen nicht weiter wachsen.

Aufgabe der vorliegenden Erfindung war es daher, ein saugfähiges Material mit hoher Absorptions- und Retentionsfähigkeit bereitzustellen.

Eine weitere Aufgabe der Erfindung bestand darin, ein saugfähiges Material mit hoher Absorptions- und Retentionsfähigkeit für Körperflüssigkeiten bereitzustellen. Unter "Körperflüssigkeiten" wird hier, wie in der vorliegenden Anmeldung und den Patentansprüchen, Schweiß, Schleim, Blut, Urin, Fäkalabsonderungen und dergleichen verstanden.

Eine weitere Aufgabe der Erfindung war es, saugfähige Materialien mit hoher Absorptions- und Retentionsfähigkeit bereitzustellen, die unter biologischen Bedingungen abbaubar sind, d. h. beispielsweise ohne Chemikalienzusatz und/oder unter Umgebungsbedingungen kompostiert oder sogar humifiziert werden können und die damit nicht in erheblichem Maße zum Müllvolumen beitragen.

Eine weitere Aufgabe der Erfindung bestand darin, für ein derartiges saugfähiges Material geeignete Verwendungen anzugeben und Anordnungen bereitzustellen, die ein solches saugfähiges Material umfassen.

Überraschenderweise wurde gefunden, daß man saugfähige Materialien mit hoher Absorptions- und Retentionsfähigkeit für Körperflüssigkeiten bereitstellen kann, wenn man aus Materialien natürlicher, insbesondere pflanzlicher Herkunft hergestellte Umhüllungen mit ebenfalls aus natürlichen Quellen, insbesondere pflanzlichen Materialien, gewonnene saugfähigen Materialien kombiniert und in diese in hohem Maße Flüssigkeiten absorbierende chemische Substanzen integriert, die aufgrund ihrer chemischen Struktur einem Abbau unter biologischen Bedingungen zugänglich sind. Überraschend wurde weiterhin gefunden, daß solche Materialien in ihrer Saugfähigkeit und Flüssigkeitsretention den bisher bekannten, synthetischen Materialien nicht oder nur unwesentlich nachstehen. Der gegebenenfalls auftretende Nachteil einer geringeren Säugfähigkeit wird jedoch durch die verbesserte biologische Abbaubarkeit bzw. Kompostierbarkeit weit überkompensiert.

Die Erfindung betrifft ein saugfähiges Material aus einer oder mehreren flüssigkeitsdurchlässigen Schichten und einem Saugkörper mit hoher Absorptions- und Retentionskapazität für wäßrige Flüssigkeiten, gekennzeichnet durch eine Absorptionskapazität von mindestens 8 g/g, eine Retentionskapazität von mindestens 6 g/g und einen Aufbau aus
(a) einer Umhüllung aus einem vliesartigen Stoff aus einem organischen Polymer;
(b) einer Tissue-Zwischenlage aus einem organischen Polymer;
(c) einem aus Zellstoff mit einem spezifischen Volumen von mindestens 15 cm³/g und einem Absorptionsvermögen von mindestens 8 g/g und einem Absorbens für wäßrige Flüssigkeiten mit einer Teilchengröße von 80 bis 1.000 µm und einem Schüttvolumen von mindestens 300 kg/m³ bestehenden Saugkörper; und
(d) einem die Umhüllung stabilisierenden Binder.

Die Erfindung betrifft auch die Verwendung eines derartigen saugfähigen Materials für die Herstellung saugfähiger Formkörper, Einlagen und Unterlagen im Säuglingspflege-, Inkontinenz- und persönlichen Hygienebereich.

Die Erfindung betrifft auch Anordnungen, die ein oben genanntes saugfähiges Material in Kombination mit einer flüssigkeitsdichten Umhüllung umfassen.

Bevorzugte Auführungsformen der Erfindung ergeben sich aus den Unteransprüchen.

Die Erfindung wird nunmehr anhand der beigefügten Figuren erläutert.

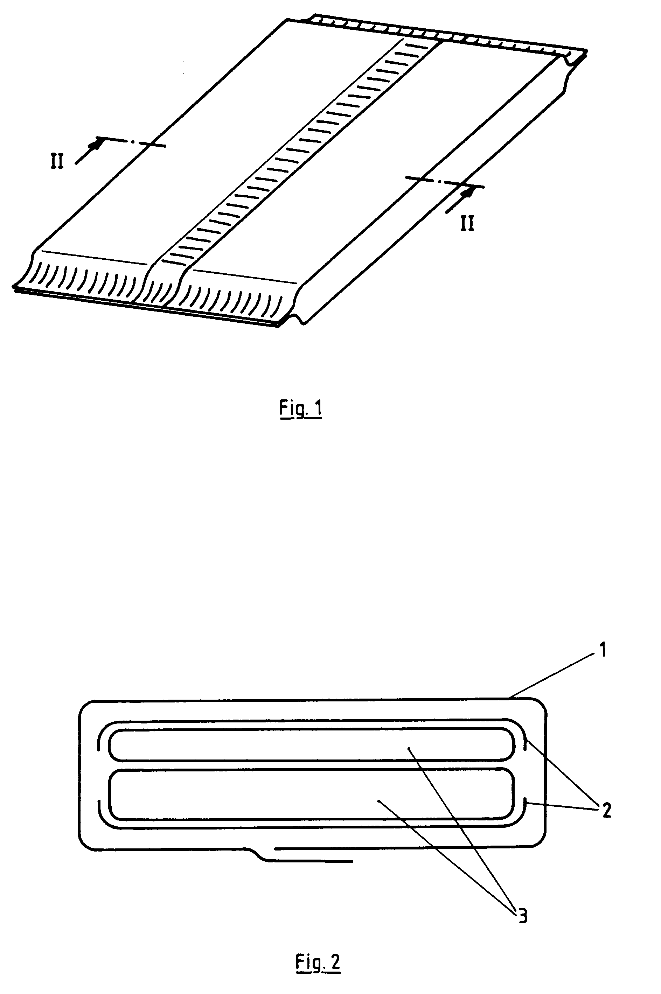

Figur 1 zeigt eine Gesamtansicht einer bevorzugten Ausführungsform des saugfähigen Materials gemäß der Erfindung im gebrauchsfertigen Zustand, ohne die Erfindung auf diese Ausführungsform zu beschränken.

Figur 2 zeigt einen Querschnitt durch eine bevorzugte Ausführungsform des saugfähigen Materials der Erfindung, ohne daß die Erfindung notwendigerweise alle Einzelbestandteile enthalten muß, die in dieser Figur gezeigt sind.

Das saugfähige Material gemäß der Erfindung ist aus einer oder mehreren flüssigkeitsdurchlässigen bzw. Flüssigkeit absorbierenden Schichten aufgebaut. Als Flüssigkeiten werden in erster Linie diejenigen Körperflüssigkeiten verstanden, die bereits oben im einzelnen beispielhaft abgegeben wurden. Sinn und Zweck der durchlässigen Schichten liegt darin, einen möglichst guten Durchtritt bzw. eine Verteilung der genannten Flüssigkeiten in das Innere des erfindungsgemäßen saugfähigen Materials zu ermöglichen und damit zu erreichen, daß die Oberfläche des saugfähigen Materials nicht in feuchtem Zustand mit der Haut in Verbindung kommt. Dies hat aufgrund der Inhaltsstoffe der genannten Flüssigkeiten häufig ein Wundwerden der entsprechenden Hautpartien zur Folge und sollte daher vermieden werden. Im Inneren des saugfähigen Materials werden die genannten Flüssigkeiten absorbiert.

Ein weiterer Bestandteil des erfindungsgemäßen saugfähigen Materials ist ein im Inneren des Materials angeordneter, von der Umgebung durch mindestens eine oben beschriebene flüssigkeitsdurchlässige Schicht getrennter Saugkörper. Dieser weist eine hohe Absorptions- und Retentionskapazität für wäßrige Flüssigkeiten, insbesondere für die oben beispielhaft angegebenen Körperflüssigkeiten, auf. Erfindungsgemäß hat das Material des Saugkörpers eine Absorptionskapazität für wäßrige Flüssigkeiten bei mindestens 8 g/g, bevorzugt im Bereich von 8 bis 14 g/g, besonders bevorzugt von 10 bis 12 g/g.

Ein weiteres charkateristisches Merkmal des Saugkörpers ist sein Rückhaltevermögen (Retentionskapazität) für die genannten wäßrigen Flüssigkeiten. Dieses liegt erfindungsgemäß bei mindestens 6 g/g, bevorzugt im Bereich von 12 bis 40 g/g, besonders bevorzugt von 18 bis 30 g/g. Der erfindungsgemäße Vorteil der genannten Werte der Absorptions- und Retentionskapazität ist darin zu sehen, daß wäßrige Flüssigkeiten innerhalb kurzer Zeit aufgenommen werden und aufgrund der chemischen Struktur der beteiligten Materialien auch gehalten und nicht wieder an die Umgebung abgegeben werden. Dies kann beispielsweise dadurch geschehen, daß ein Teil der Bestandteile des Saugkörpers aufgrund seiner chemischen Struktur unter Flüssigkeitsaufnahme aufquillt und damit eine Abgabe der Flüssigkeit an die Umgebung verhindert. Der tatsächliche Absorptions- und Retentionsmechanismus ist jedoch nicht auf diese chemische Erscheinung beschränkt.

Erfindungsgemäß ist der Saugkörper (3) des saugfähigen Materials aus Zellstoff ("Fluff") und einem Absorbens für wäßrige Flüssigkeiten aufgebaut. Unter Absorbens für wäßrige Flüssigkeiten werden Stoffe verstanden, die solche Flüssigkeiten absorbieren und einen relativ hohen Prozentsatz derartiger Flüssigkeiten, gegebenenfalls auch unter Druck, zu halten vermögen. Der Saugkörper (3) macht erfindungsgemäß etwa 80 bis 90 % (Gewicht) des saugfähigen Materials aus, bevorzugt 85 bis 87 %. Innerhalb des Saugkörpers liegt dabei das Gewichtsverhältnis der beiden Komponenten Zellstoff und Absorbens im Bereich von 9 : 1 bis 14 : 1, wobei das Verhältnis von etwa 12 : 1 bevorzugt ist.

Der Zellstoff weist folgende Eigenschaften auf: Der Aufgabenstellung der vorliegenden Erfindung entsprechend, unter biologischen Bedingungen abbaubare, beispielsweise kompostierbare oder humifizierbare saugfäige Materialien bereitzustellen, wird ausschließlich Zellstoff verwendet, der keine die Umwelt schädigenden Komponenten enthält. Insbesondere ist erfindungsgemäß Material bevorzugt, das unter Bedingungen hergestellt wird, die in geringstmöglichem Umfang auf die Umwelt schädigend einwirken. Beispielsweise wird in einer besonders bevorzugten Ausführungsform Zellstoff verwendet, der ohne Verwendung von Chlor gebleicht wurde oder einem Bleichvorgang nicht ausgesetzt wurde.

Das Zellstoffmaterial sollte ein hohes Benetzungsvermögen aufweisen. Das spezifische Volumen des erfindungsgemäß verwendeten Zellstoffs liegt bei mindestens 15 cm³/g, bevorzugt bei mindestens 20 cm³/g. Das Absorptionsvermögen des Zellstoffs des Saugkörpers liegt bei mindestens 8 g/g, bevorzugt bei 8 bis 12 g/g. Der Fachmann kennt weitere Materialien für gleiche Zwecke, die ebenfalls ohne Unterschied verwendet werden können, solange das Ziel der vorliegenden Erfindung nicht gefährdet wird, die saugfähigen Materialien unter biologischen Bedingungen kompostieren zu können.

Das erfindungsgemäß in dem saugfähigen Material verwendete Absorbens für wäßrige Flüssigkeiten hat eine Teilchengröße im Bereich von 80 bis 1.000 mm, bevorzugt von 200 bis 600 mm, besonders bevorzugt bei etwa 400 mm. Es weist darüberhinaus in bevorzugten Ausführungsformen ein Schüttvolumen von mindestens 300 kg/m³, bevorzugt von 350 bis 450 kg/m³, auf.

In einer bevorzugten Ausführungsform der Erfindung ist das Absorbens gewählt aus der Gruppe hochmolekularer wasserlöslicher Polyelektrolyten auf Polyacrylatbasis, Carboxymethylcellulose und gepfropfte Stärke. Derartige Materialien sind im Stand der Technik als solche bekannt und können im Handel käuflich erworben werden. Beispielhaft seien in diesem Zusammenhang die sogenannten Superabsorbents genannt, die sehr starke Polyelektrolyten sind und aufgrund ihres hohen Molekulargewichts nicht in Wasser löslich sind. Die erfindungsgemäß verwendbaren Superabsorbents weisen ein ausgesprochen hohes Wasseranzugsvermögen auf. Sie können ausschließlich natürlichen Produkten entstammen oder auch chemisch modifizierte Produkte natürlichen Ursprungs sein. Erfindungsgemäß bevorzugte Beispiele sind Carboxymethylcellulose, Polyacrylate und gepfropfte Stärke, insbesondere mit Acrylgruppen gepfropfte Stärke. Die Acrylatgruppen können dabei mit geeigneten, physiologisch unbedenklichen und für das biologische Abbauvermögen unschädlichen Metallionen, bevorzugt Alkalimetall- und/oder Erdalkalimetallionen neutralisiert sein. Geeignete Produkte sind beispielsweise unter den Handelsnamen Akucell ^{R} SW von der Firma Akzo oder unter dem Produktnamen Drystar ^{R} H 20 und H 40 von der Firma Starchem oder unter dem Produktnamen Sanwet ^{R} M 5000 S der Firma Hoechst AG bekannt.

In besonders bevorzugten Ausführungsformen weisen diese Produkte beispielsweise einen pH-Wert (0,5 % in 1 %iger NaCl-Lösung) im neutralen bis leicht sauren pH-Wertbereich, beispielsweise bei pH 6 auf, und verfügen über eine Absorptionskapazität (Zentrifugation) im Bereich von 25 bis 50 g/g, bevorzugt 30 bis 45 g/g, wobei als absorbierte Flüssigkeit eine wäßrige Flüssigkeit wie beispielsweise Urin, für Testzwecke insbesondere synthetischer Urin, verwendet werden kann. Die Retentionskapazität für die gleiche wäßrige Flüssigkeit liegt in diesem bevorzugten Fall im Bereich von 10 bis 40 g/g, bevorzugt im Bereich von 15 bis 30 g/g. Derartige Materialien kommen in Form eines freifließenden weißen Pulvergranulats in den Handel und sind auch nach der Flüssigkeitsaufnahme, d. h. nach dem Aufquellen, körnchenartige Gelteilchen.

Zum erfindungsgemäßen Aufbau des saugfähigen Materials gehört außer dem Saugkörper (3) auch eine Umhüllung (1) aus einem vliesartigen Stoff aus einem organischen Polymer ("Coverstock"). In einer bevorzugten Ausführungsform der Erfindung besteht die Umhüllung (1) aus einem Vlies aus einem organischen Polymer pflanzlichen Ursprungs. Besonders bevorzugt werden Vliese (non-wovens) aus baumwollartigen Materialien, die beispielsweise zwischen 5 und 8 Gew.-%, bevorzugt zwischen 6 und 7 Gew.-%, des gesamten Materials ausmachen. Derartige Materialien sind im Handel in verschiedenen Ausführungsformen erhältlich. Außer Baumwolle können auch andere Materialien, wie beispielsweise Zellwolle/Viskose, in Frage kommen. Geeignet sind auch unter der Bezeichnung Rayon ^{R} erhältliche Viskosefasern, solange sie unter biologischen Bedingungen abbaubar sind. Solche Materialien (non-wovens) sind beispielsweise von der Firma Lohmann unter dem Namen Paraprint OL 25 P 20 im Handel. Selbstverständlich sind jedoch auch Materialien aus anderen organischen Polymeren, insbesondere organischen Polymeren pflanzlichen Ursprungs, verwendbar.

In einer weiteren bevorzugten Ausführungsform der Erfindung ist die Umhüllung (1) so angeordnet, daß sie das saugfähige Material ringsum einschließt. Mittels eines geeigneten Binders wird diese Anordnung in der Weise stabilisiert, daß ein Austreten des Saugkörpermaterials, d. h. des Zellstoffs (Cellulose) und/oder des Absorbens für wäßrige Flüssigkeiten, nicht erfolgen kann. Diese Ausführungsform ist deswegen als besonders vorteilhaft anzusehen, weil dadurch verhindert wird, daß vor Gebrauch, während des Gebrauchs oder nach dem Gebrauch Inhaltsstoffe des erfindungsgemäßen saugfähigen Materials aus der Gesamtanordnung austreten und damit den Gebrauchswert des Materials mindern können.

In einer weiteren bevorzugten Ausführungsform der Erfindung tritt eine Stabilisierung des Superabsorbers im Zellstoff zusätzlich zur Verwendung eines geeigneten Binders dadurch ein, daß das saugfähige Material mit einer geeigneten Prägung, beispielsweise einer an sich aus dem Stand der Technik bekannten Rautenprägung, versehen wird. Diese komprimiert das saugfähige Material in der Weise, daß das Volumen des Zellstoffs gezielt vermindert und die Partikel des Superabsorbers im Saugkörper mehr oder weniger fixiert werden. Ein Austreten des Superabsorbers erfolgt dadurch nicht mehr. Ein weiterer vorteilhafter Effekt, der durch die Prägung erzielt wird, geht dahin, daß sich durch die Prägung "Kanäle" bilden, die ein relativ schnelles Weiterleiten der eintretenden Flüssigkeit vom Eintrittspunkt in den umgebenden Bereich ermöglichen und dadurch eine gleichmäßige Verteilung in einem größeren Bereich des Saugkörpers ermöglichen. Das Aufbringen der Prägung erfolgt in an sich aus dem Stand der Technik bekannter Weise, beispielsweise dadurch, daß man das saugfähige Material (Zellstoff (Cellulose) zusammen mit dem Superabsorber und dem Binder) durch geeignete Walzen laufen läßt.

In einer weiteren bevorzugten Ausführungsform der Erfindung umfaßt der Binder Verbindungen aus der Gruppe der EVA-Polymeren oder damit verwandter Polymerer, gegebenenfalls mit weiteren polymerisierbaren Olefinmonomeren. Geeignet ist in diesem Zusammenhang, ohne die Erfindung darauf zu beschränken, ein Binder namens H 331 plus der Firma Ecomelt oder ein Binder namens Dispomelt 217 der Firma National Starch & Chemical B. V..

Ein weiterer Bestandteil des erfindungsgemäßen saugfähigen Materials ist eine Tissuezwischenlage (2) aus einem organischen Polymer, die den Zweck haben soll, den Saugkörper zu fixieren und die aufzunehmende Feuchtigkeit zu verteilen. In einer bevorzugten Ausführungsform besteht diese Zwischenlage ebenfalls Cellulose bzw. modifizierter Cellulose, also in weitestem Sinne Zellstoffmaterialien und macht zwischen 3 und 6 Gew.-%, bevorzugt etwa 4 bis 5 Gew.-%, des gesamten Materials aus.

Durch die beschriebene Anordnung wird ein saugfähiges Material bereitgestellt, das im Vergleich zu Materialien aus dem Stand der Technik eine zumindest gleichgute Absorptions- und Retentionskapazität für wäßrige Flüssigkeiten aufweist. Entscheidendes Ziel der vorliegenden Erfindung war es jedoch, saugfähige Materialien bereitzustellen, die einem Abbau unter biologischen Bedingungen zugänglich sind. Dieses Ziel wird mit dem erfindungsgemäßen saugfähigen Material erreicht. Aufgrund seines Aufbaus ausschließlich aus organischen Polymeren natürlichen Ursprungs bzw. von solchen Polymeren durch einfache chemische Reaktionen abgeleiteten Polymeren entsteht ein für die Umwelt völlig unbedenkliches, durch natürliche chemische Reaktionen abbaubares saugfähiges Material. Dieses kann aufgrund seiner Ähnlichkeit bzw. Identität mit natürlichen Materialien von bei der Kompostierung bzw. Humifizierung tätigen Mikroorganismen unter biologischen Bedingungen und ohne Zusatz irgendwelcher Chemikalien zumindest weitgehend abgebaut werden. Dadurch wird ermöglicht, die Entsorgung der Materialien so anzulegen, daß die nichtverrottbaren Müllenmengen nicht weiter ansteigen.

In den Fällen, in denen ein biologischer Abbau unter dem Einfluß der bei der Kompostierung bzw. Humifizierung tätigen Mikroorganismen unter biologischen Bedingungen nicht vollständig erfolgen kann und die Kompost- bzw. Humusmaterialien später in den Boden eingearbeitet werden, können Rückstände an Superabsorber oder Binder, die nicht vollständig abgebaut wurden, als Mittel zur Auflockerung des Bodens einerseits und zur Verbesserung des Flüssigkeitshaltevermögens andererseits wirken. Diese Materialien verbleiben über relativ lange Zeit im Boden und tragen dazu bei, daß die Bodenkrume unter Feuchtigkeitseinfluß locker bleibt und Wasser nicht unmittelbar durchsickert, sondern über gewisse Zeit in der entsprechenden Bodenschicht gehalten wird. Zu berücksichtigen ist in diesem Fall, daß der Anteil des humifizierten Materials, der aus den erfindungsgemäßen saugfähigen Materialien stammt, relativ klein ist und sich damit in dem Bereich bewegt, der gerade für eine Verbesserung der physikalischen Bodeneigenschaften ausreicht.

Erfindungsgemäß wird das oben beschriebene saugfähige Material für die Herstellung saugfähiger Formkörper, Einlagen und Unterlagen im Säuglingspflege-, Inkontinenz- und persönlichen Hygienebereich verwendet. Beispielhaft seien dafür Einlagen für Babywindeln, Inkontinenzeinlagen oder -unterlagen und Damenbinden genannt. Im Bereich der Verwendung von Einlagen für Babywindeln läßt sich mit dem erfindungsgemäßen saugfähigen Material insbesondere die angestrebte "Zwei-Stück-System"-Lösung erfolgreich verwirklichen. Der Vorteil bei der Verwendung der erfindungsgemäßen Materialien liegt nicht nur in deren zufriedenstellender Absorptions- und Retentionskapazität für wäßrige Flüssigkeiten, sondern auch in ihrer biologischen Abbaubarkeit und damit ihrer umweltgerechten Entsorgung.

Die Erfindung betrifft auch eine Anordnung, die ein saugfähiges Material, wie es oben im einzelnen beschrieben wurde, in Kombination mit einer flüssigkeitsdichten Umhüllung umfaßt. Diese Umhüllung sorgt für den gegen Flüssigkeitsaustritt sicheren Sitz des saugfähigen Materials gemäß der Erfindung. Sie kann beispielsweise ein Windelhöschen sein, das im Hinblick auf den der Erfindung zugrundeliegenden Grundsatz, Rohstoffe einzusparen, ein mehrfach (gegebenenfalls nach Waschen) wiederverwendbares Höschen aus einem flüssigkeitsdichten Material sein kann, ohne hierauf beschränkt zu sein. Bewährt haben sich beispielsweise unter dem Namen Darlexx^{R} erhältliche, in Höschenform geschnittene Umhüllungen. Das Material ist beispielsweise in der US-PS 4,761,321 beschrieben. Flüssigkeitsdichte Umhüllungen sind beispielsweise auch Unterlagen für Inkontinenzmaterialien auf geeigneter Basis, beispielsweise auf Basis des Materials Darlexx ^{R}.

In jüngerer Zeit wurde bei der Herstellung von Windeln den physiologischen Gegebenheiten dadurch besonders Rechnung getragen, daß man unterschiedliche Windeltypen für Jungen und Mädchen entwikkelte. Nachteil dieser Lösung war es in jedem Fall, daß für jedes Geschlecht ein unterschiedlicher Windeltyp bereitgestellt werden mußte.

Die Erfindungsgemäße Zwei-Stück-System-Lösung vereinfacht es in verblüffender Weise, für Jungen und Mädchen unterschiedliche saugfähige Materialien zur Verfügung zu stellen. Dies geschieht in einfacher Weise dadurch, daß man beispielsweise etwa 80 % der Gesamtmenge an Superabsorber in einem Volumen saugfähigen Materials verteilt, das etwa zwei Drittel der Oberfläche entspricht. Dieser Bereich des saugfähigen Materials ist naturgemäß besser saugfähig als der Bereich, in dem die Gesamtmenge an Superabsorber deutlich geringer ist. Durch eine von außen erkennbare Markierung des saugfähigen Materials kann man dann dieses in der Windelhose bzw. der Hygieneeinlage oder Inkontinenzeinlage so anordnen, daß dem geschlechtsspezifischen Flüssigkeitsanfall Rechnung getragen wird.

Die Erfindung wird durch das nachfolgende Beispiel näher erläutert.

### Beispiel

Es wurde mit 20 Müttern mit Kleinkindern ein Test durchgeführt, in dem die Babys unter Verwendung der erfindungsgemäßen Anordnungen, d. h. mit den saugfähigen Materialien und einer flüssigkeitsdichten Umhüllung in Form einer Windelhose gemäß der Erfindung gewickelt wurden.

Bei den Kleinkindern traten in einem Zeitraum von 3 Monaten des Tests keinerlei Hautreizungen oder sonstige Beschwerden auf. Die Funktionstüchtigkeit des Zwei-Stück-Systems bewährte sich auch im Hinblick auf die Tatsache, daß ein saugfähiges Material sowohl für Jungen als auch für Mädchen verwendet werden konnte.

Die Mütter bemerkten auch, daß die Geruchsbelästigung im Vergleich zu herkömmlichen Babywindeln geringer war. Offenbar ist die Windelhose durchlässiger für Luft, verglichen mit der bei Babyfertigwindeln sonst üblichen Plastikfolie. Dies minimiert die Geruchsbildung.

Die gebrauchten saugfähigen Materialien wurden getrennt gesammelt und einmal wöchentlich bei den Haushalten abgeholt. Sie wurden dann auf einer Kompostierungsanlage unter Aufsicht geschreddert und mit anderen kompostierbaren Garten- und Küchenabfällen kompostiert. Danach wurden sie von ortsansässigen Landwirten zur Bodenverbesserung auf landwirtschaftliche Flächen ausgebracht.

Die Bauern waren mit den Bodenverbesserungsergebnisse zufrieden. Insbesondere die erhöhte Feuchtigkeits-Speicherkapazität des Bodens durch den nicht mikrobiologisch abgebauten Superabsorber wurde positiv hervorgehoben.

## Patentansprüche

1. Saugfähiges Material aus einer oder mehreren flüssigkeitsdurchlässigen Schicht(en) und einem Saugkörper mit hoher Absorptions- und Retentionskapazität für wäßrige Flüssigkeiten, gekennzeichnet durch eine Absorptionskapazität von mindestens 8 g/g und eine Retentionskapazität von mindestens 6 g/g sowie einen Aufbau aus
(a) einer Umhüllung (1) aus einem vliesartigen Stoff aus einem organischen Polymer;
(b) einer Tissuezwischenlage (2) aus einem organischen Polymer;
(c) einem aus Zellstoff mit einem spezifischen Volumen von mindestens 15 cm³/g und einem Absorptionsvermögen von mindestens 8 g und einem Absorbens für wäßrige Flüssigkeiten mit einer Teilchengröße von 80 bis 1.000 µm und einem Schüttvolumen von mindestens 300 kg/m³ bestehenden Saugkörper (3); und
(d) einem die Umhüllung (1) stabilisierenden Binder.

2. Saugfähiges Material nach Anspruch 1, worin die Umhüllung (1) aus einem Vlies aus einem organischen Polymer pflanzlichen Ursprungs besteht.

3. Saugfähiges Material nach Anspruch 2, worin die Umhüllung (1) ein Baumwollvlies oder ein Zellwolle-/Viskosevlies ist.

4. Saugfähiges Material nach einem oder mehreren der Ansprüche 1 bis 3, worin die Umhüllung (1) die ringsumverlaufende Außenschicht des Material darstellt, die mittels des Binders in der Weise stabilisiert ist, daß ein Austreten des Saugkörpermaterials nicht erfolgen kann.

5. Saugfähiges Material nach einem oder mehreren der Ansprüche 1 bis 4, worin der Binder gewählt ist aus der Gruppe der EVA-Polymeren und damit verwandter Polymerer, gegebenenfalls in Verbindung mit weiteren polymerisierbaren Olefinmonomeren.

6. Saugfähiges Material nach einem oder mehreren der Ansprüche 1 bis 5, worin die Tissuezwischenlage (2) aus Zellstoff besteht.

7. Saugfähiges Material nach einem oder mehreren der Ansprüche 1 bis 6, worin der Saugkörper (3) aus Zellstoff und Absorbens im Gewichtsverhältnis 9 : 1 bis 14 : 1, besonders bevorzugt im Verhältnis von etwa 12 : 1, besteht.

8. Saugfähiges Material nach einem oder mehreren der Ansprüche 1 bis 7, worin das Absorbens gewählt ist aus der Gruppe hochmolekularer wasserlöslicher Polyelektrolyten auf Polyacrylatbasis, Carboxymethylcellulose und gepfropfte Stärke, bevorzugt Acrylat-gepfropfte Stärke, besonders bevorzugt mit physiologisch unbedenklichen Alkali- oder Erdalkaliionen neutralisierte, Acrylat-gepfropfte Stärke.

9. Saugfähiges Material nach einem oder mehreren der Ansprüche 1 bis 8, worin das Absorbens eine Absorptionskapazität im Bereich von 25 bis 50 g/g hat.

10. Saugfähiges Material nach einem oder mehreren der Ansprüche 1 bis 8, worin das Absorbens eine Retentionskapazität im Bereich von 10 bis 40 g/g hat.

11. Saugfähiges Material nach einem oder mehreren der Ansprüche 1 bis 10, worin der Zellstoff des Saugkörpers (3) eine Absorptionskapazität im Bereich von 8 bis 12 g/g hat.

12. Verwendung des saugfähigen Materials nach einem oder mehreren der Ansprüche 1 bis 11 für die Herstellung saugfähiger Formkörper, Einlagen und Unterlagen im Säuglingspflege-, Inkontinenz- und persönlichen Hygienebereich.

13. Verwendung nach Anspruch 12 für Einlagen von Babywindeln.

14. Verwendung nach Anspruch 12 für Inkontinenzeinlagen.

15. Verwendung nach Anspruch 12 für Damenbinden.

16. Anordnung, umfassend ein saugfähiges Material nach einem oder mehreren der Ansprüche 1 bis 11 in Kombination mit einer flüssigkeitsdichten Umhüllung.

17. Anordnung nach Anspruch 16, worin die flüssigkeitsdichte Umhüllung ein Windelhöschen ist.
